# EUROPEAN PATENT APPLICATION

(11) **EP 4 813 288 A1**
(43) Date of publication of application: **30.09.2026**
(21) Application number: 25755061.6
(22) Date of filing: 13.01.2025
(51) Int. Cl.: A61B 5/021, A61B 5/00, A61B 5/024, A61B 5/02

(54) **METHOD FOR MEASURING BLOOD PRESSURE AND ELECTRONIC DEVICE SUPPORTING SAME**

(30) Priority: 13.02.2024 KR 20240020256; 05.04.2024 KR 20240046910
(71) Applicant: Samsung Electronics Co., Ltd., Suwon-si, Gyeonggi-do 16677 (KR)
(72) Inventor: HAN, Jonghee, Suwon-si Gyeonggi-do 16677 (KR); JANG, Hyunwoo, Suwon-si Gyeonggi-do 16677 (KR); CHUNG, Gihsung, Suwon-si Gyeonggi-do 16677 (KR)
(74) Representative: Gulde & Partner
(86) International application number: PCT/KR2025/000709
(87) International publication number: WO 2025/173939

(57) **Abstract**

An electronic device according to one embodiment may comprise a communication module and at least one processor. The at least one processor may be configured to receive a first PPG signal obtained by a first wearable device from the first wearable device and receive a second PPG signal from a second wearable device through a communication circuit. The first wearable device may be worn at a first location on a user and the second wearable device may be worn at a second location on the user. The at least one processor may be configured to obtain a pulse transit time (PTT) for which pulse waves are transmitted from the first location to the second location, on the basis of the first PPG signal and the second PPG signal.. The at least one processor may be configured to obtain the distance between the first wearable device and the second wearable device. The at least one processor may be configured to obtain a pulse wave velocity (PWV) at which pulse waves are transmitted, on the basis of the distance and the PTT. The at least one processor may be configured to obtain a first blood pressure by using a pulse wave analysis (PWA) method on the basis of the first PPG signal and/or the second PPG signal. The at least one processor may be configured to obtain a blood vessel characteristic value related to the characteristics of blood vessels, on the basis of the PWV and the first blood pressure. The at least one processor may be configured to obtain a blood pressure correction value for correction of a second blood pressure that is to be obtained, on the basis of the blood vessel characteristic value.

## Description

### [TECHNICAL FIELD]

The disclosure relates to a method of measuring a blood pressure and an electronic device supporting same.

### [BACKGROUND ART]

Electronic devices are evolving in various forms for user convenience and are being miniaturized so that they are conveniently portable for users.

Recently, with the growing interest in health, electronic devices have been measuring biometric signals related to the human body and providing biometric information, based on the measured biometric signals. For example, an electronic device (e.g., a wearable device) may obtain a photoplethysmogram (PPG) signal through an optical sensor (e.g., a photoplethysmogram (PPG) sensor) for obtaining a biometric signal, and measure a blood pressure, based on the obtained PPG signal.

The above information is presented as background information only to assist with an understanding of the disclosure. No determination has been made, and no assertion is made, as to whether any of the above might be applicable as prior art with regard to the disclosure.

### [DETAILED DESCRIPTION OF THE INVENTION]

### [Technical Solution]

An electronic device (e.g., a wearable device) may correct a blood pressure (e.g., blood pressure value) measured based on a PPG signal by using a blood pressure measured using a blood pressure monitor, thereby calculating a final blood pressure. For example, the electronic device may measure a blood pressure (hereinafter, this is referred to as a "blood pressure measured based on a PPG signal") by using a pulse wave analysis (PWA) method, based on a PPG signal obtained through a PPG sensor. However, a user's blood vessel state may change over time. Accordingly, the electronic device may periodically calculate a correction value (hereinafter, this is referred to as a "correction value") for correcting the blood pressure measured based on the PPG signal, by using a blood pressure (hereinafter, this is referred to as a "reference blood pressure") measured using a blood pressure monitor (e.g., a blood pressure monitor using a cuff). The electronic device may, after the correction value is calculated, correct the blood pressure measured based on the PPG signal by using the correction value (e.g., add the correction value to the blood pressure measured based on the PPG signal), thereby calculating a final blood pressure (e.g., a blood pressure to be provided to the user).

However, measuring a reference blood pressure by using a blood pressure monitor and periodically performing of an operation of calculating the correction value by using the measured reference blood pressure may be inconvenient for the user. In addition, measurement of a more precise blood pressure may be required.

Aspects of the disclosure are to address at least the above-mentioned problems and/or disadvantages and to provide at least the advantages described below. Accordingly, an aspect of the disclosure is to provide a method of measuring a blood pressure and an electronic device supporting same, wherein an operation of measuring a blood pressure is performed in cooperation with multiple wearable devices, so that provision of a more precise blood pressure is possible without periodically performing the operation of calculating the correction value.

Additional aspects will be set forth in part in the description which follows and, in part, will be apparent from the description, or may be learned by practice of the presented embodiments.

An electronic device according to an embodiment may include communication circuitry and at least one processor. The at least one processor may be configured to receive a first PPG signal obtained by a first wearable device from the first wearable device via the communication circuitry and receive a second PPG signal from a second wearable device via the communication circuitry. The first wearable device may be worn on a first position of a user and the second wearable device may be worn on a second position of the user. The at least one processor may be configured to obtain a pulse transit time (PTT) during which a pulse wave is transferred from the first position to the second position, based on the first PPG signal and the second PPG signal. The at least one processor may be configured to obtain a distance between the first wearable device and the second wearable device. The at least one processor may be configured to obtain a pulse wave velocity (PWV) at which the pulse wave is transferred, based on the distance and the PTT. The at least one processor may be configured to obtain a first blood pressure by using a pulse wave analysis (PWA) method, based on the first PPG signal and/or the second PPG signal. The at least one processor may be configured to obtain a vascular characteristic value related to a characteristic of a blood vessel, based on the PWV and the first blood pressure. The at least one processor may be configured to obtain a blood pressure correction value for correcting a second blood pressure to be obtained, based on the vascular characteristic value.

A method of providing a blood pressure by an electronic device according to an embodiment may include receiving a first PPG signal obtained by a first wearable device from the first wearable device via communication circuitry of the electronic device, and receiving a second PPG signal from a second wearable device via the communication circuitry. The first wearable device may be worn on a first position of a user and the second wearable device may be worn on a second position of the user. The method may include obtaining a pulse transit time (PTT) during which a pulse wave is transferred from the first position to the second position, based on the first PPG signal and the second PPG signal. The method may include obtaining a distance between the first wearable device and the second wearable device. The method may include obtaining a pulse wave velocity (PWV) at which the pulse wave is transferred, based on the distance and the PTT. The method may include obtaining a first blood pressure by using a pulse wave analysis (PWA) method, based on the first PPG signal and/or the second PPG signal. The method may include obtaining a vascular characteristic value related to a characteristic of a blood vessel, based on the PWV and the first blood pressure. The method may include obtaining a blood pressure correction value for correcting a second blood pressure to be obtained, based on the vascular characteristic value.

In an embodiment, in a non-transitory computer-readable medium for recording computer-executable instructions, the computer-executable instructions may, when executed by at least one processor of an electronic device, cause the electronic device to receive a first PPG signal obtained by a first wearable device from the first wearable device via communication circuitry of the electronic device and receive a second PPG signal from a second wearable device via the communication circuitry. The first wearable device may be worn on a first position of a user and the second wearable device may be worn on a second position of the user. The computer-executable instructions may, when executed by the at least one processor of the electronic device, cause the electronic device to obtain a pulse transit time (PTT) during which a pulse wave is from the first position to the second position, based on the first PPG signal and the second PPG signal. The computer-executable instructions may, when executed by the at least one processor of the electronic device, cause the electronic device to obtain a distance between the first wearable device and the second wearable device. The computer-executable instructions may, when executed by the at least one processor of the electronic device, cause the electronic device to obtain a pulse wave velocity (PWV) at which the pulse wave is transferred, based on the distance and the PTT. The computer-executable instructions may, when executed by the at least one processor of the electronic device, cause the electronic device to obtain a first blood pressure by using a pulse wave analysis (PWA) method, based on the first PPG signal and/or the second PPG signal. The computer-executable instructions may, when executed by the at least one processor of the electronic device, cause the electronic device to obtain a vascular characteristic value related to a characteristic of a blood vessel, based on the PWV and the first blood pressure. The computer-executable instructions may, when executed by the at least one processor of the electronic device, cause the electronic device to obtain a blood pressure correction value for correcting a second blood pressure to be obtained, based on the vascular characteristic value.

Other aspects, advantages, and salient features of the disclosure will become apparent to those skilled in the art from the following detailed description, which, taken in conjunction with the annexed drawings, discloses various embodiments of the disclosure.

### [BRIEF DESCRIPTION OF DRAWINGS]

The above and other aspects, features, and advantages of certain embodiments of the disclosure will be more apparent from the following description taken in conjunction with the accompanying drawings, in which:
FIG. 1 is a block diagram of an electronic device in a network environment according to an embodiment of the disclosure;
FIG. 2A is a perspective view of a front surface of a first wearable device according to an embodiment of the disclosure;
FIG. 2B is a perspective view of a rear surface of a first wearable device according to an embodiment of the disclosure;
FIG. 3A is a perspective view of a second wearable device according to an embodiment of the disclosure;
FIG. 3B is a sectional view of a second wearable device according to an embodiment of the disclosure;
FIG. 4 is a block diagram of an electronic device according to an embodiment of the disclosure;
FIG. 5 is a block diagram of a first wearable device according to an embodiment of the disclosure;
FIG. 6 is a block diagram of a second wearable device according to an embodiment of the disclosure;
FIG. 7 is a flowchart illustrating a method of measuring a blood pressure according to an embodiment of the disclosure;
FIG. 8 is a diagram illustrating a method of synchronizing a first wearable device and a second wearable device according to an embodiment of the disclosure;
FIG. 9 is a diagram illustrating a method of obtaining a PTT, based on a first PPG signal and a second PPG signal according to an embodiment of the disclosure;
FIG. 10 is a diagram illustrating a method of obtaining a distance between a first wearable device and a second wearable device according to an embodiment of the disclosure;
FIG. 11 is a diagram illustrating a method of obtaining a vascular characteristic value by using a PWV blood pressure model and a PWA blood pressure model according to an embodiment of the disclosure;
FIG. 12 is a diagram illustrating a method of obtaining a blood pressure correction value, based on a vascular characteristic value according to an embodiment of the disclosure; and
FIG. 13 is a flowchart illustrating a method of obtaining a correlation between a vascular characteristic value and a blood pressure correction value according to an embodiment of the disclosure.

The same reference numerals are used to represent the same elements throughout the drawings.

### [MODE FOR CARRYING OUT THE INVENTION]

The following description with reference to the accompanying drawings is provided to assist in a comprehensive understanding of various embodiments of the disclosure as defined by the claims and their equivalents. It includes various specific details to assist in that understanding but these are to be regarded as merely exemplary. Accordingly, those of ordinary skill in the art will recognize that various changes and modifications of the various embodiments described herein can be made without departing from the scope and spirit of the disclosure. In addition, descriptions of well-known functions and constructions may be omitted for clarity and conciseness.

The terms and words used in the following description and claims are not limited to the bibliographical meanings, but, are merely used by the inventor to enable a clear and consistent understanding of the disclosure. Accordingly, it should be apparent to those skilled in the art that the following description of various embodiments of the disclosure is provided for illustration purpose only and not for the purpose of limiting the disclosure as defined by the appended claims and their equivalents.

It is to be understood that the singular forms "a," "an," and "the" include plural referents unless the context clearly dictates otherwise. Thus, for example, reference to "a component surface" includes reference to one or more of such surfaces.

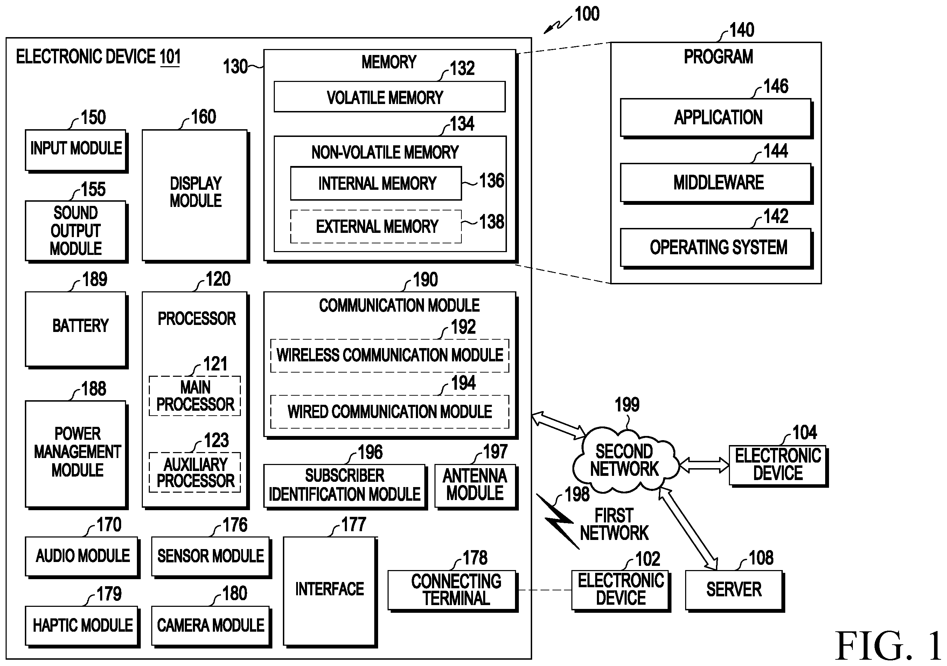

FIG. 1 is a block diagram illustrating an electronic device 101 in a network environment 100 according to an embodiment of the disclosure.

Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that an embodiment of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with an embodiment of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

An embodiment as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to an embodiment of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to an embodiment, each component (e.g., a module or a program) of the above-described components may include a single entity or multiple entities, and some of the multiple entities may be separately disposed in different components. According to an embodiment, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to an embodiment, operations performed by the module, the program, or another component may be carried out sequentially, in parallel, repeatedly, or heuristically, or one or more of the operations may be executed in a different order or omitted, or one or more other operations may be added.

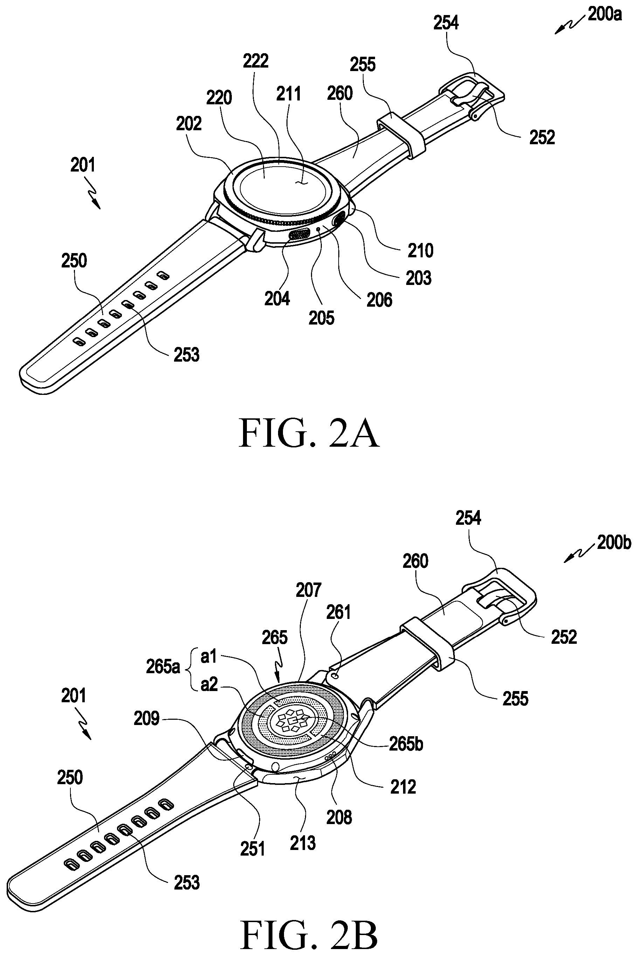

FIG. 2A is a perspective view 200a of a front surface of a first wearable device 201 according to an embodiment of the disclosure.

FIG. 2B is a perspective view 200b of a rear surface of the first wearable device 201 according to an embodiment of the disclosure.

Referring to FIGS. 2A and 2B, the first wearable device 201 according to an embodiment may include a housing 210 including a first surface (or front surface) 211, a second surface (or rear surface) 211b, and a lateral surface 213 surrounding a space between the first surface 211 and the second surface 212, and wearing members 250 and 260 connected to at least a part of the housing 210 and configured to detachably attach the first wearable device 201 to a part (e.g., wrist or ankle) of a user's body. In another embodiment of the disclosure, the housing 210 may indicate a structure configuring a part of the first surface 211, the second surface 212, and the lateral surface 213 illustrated in FIGS. 2A and 2B. According to an embodiment of the disclosure, the first surface 211 may be configured by a front plate 222 (e.g., a polymer plate or a glass plate including various coating layers), at least a part of which is substantially transparent. The second surface 212 may be configured by a rear plate 207 that is substantially opaque. In an embodiment of the disclosure, when a sensor module 265 (e.g., the sensor module 176 in FIG. 1) disposed at the second surface 212 of the first wearable device 201 is included, the rear plate 207 may at least partially include a transparent area.

The rear plate 207 may be made of, for example, coated or colored glass, ceramic, polymer, metal (e.g., aluminum, stainless steel (STS), or magnesium), or a combination of two or more of the materials. The lateral surface 213 may be configured by a lateral bezel (or a "lateral member") 206 that is coupled to the front plate 222 and the rear plate 207 and includes metal and/or polymer. In an embodiment of the disclosure, the rear plate 207 and the lateral bezel structure 206 may be integrally configured, and may include an identical material (e.g., a metal material, such as aluminum). The wearing members 250 and 260 may be made of various materials and forms. For example, the wearing members may be made of fabric, leather, rubber, urethane, metal, ceramic, or a combination of two or more of the above materials to be one piece or multiple unit links movable with respect to each other.

According to an embodiment of the disclosure, the first wearable device 201 may include at least one of a display 220 (e.g., the display module 160 in FIG. 1), an audio module 205 and 208 (e.g., the audio module 170 in FIG. 1), the sensor module 265 (e.g., the sensor module 176 in FIG. 1), key input devices 202, 203, and 204 (e.g., the input module 150 in FIG. 1), and a connector hole 209. In an embodiment of the disclosure, the first wearable device 201 may omit at least one (e.g., the key input devices 202, 203, and 204, the connector hole 209, or the sensor module 265) of the elements or additionally include another element.

According to an embodiment of the disclosure, the first wearable device 201 may include multiple electrodes for measurement of a biometric signal, and at least one electrode among the multiple electrodes may be disposed at least one position among a position of the key input device 202, 203, or 204, a position of the lateral bezel 206, and a position of the display 220 or the housing 210. The wheel key 202 among the key input devices may include a rotary bezel.

The display 220 may be, for example, exposed through a considerable part of the front plate 222. The display 220 may have a shape corresponding to a shape of the front plate 222, and may have various shapes, such as a circle, an oval, or a polygon. The display 220 may be coupled to or disposed to be adjacent to a touch detection circuit, a pressure sensor capable of measuring the strength (pressure) of touch, and/or a fingerprint sensor.

According to an embodiment of the disclosure, the display 220 may include at least one transparent electrode for biometric signal measurement among the multiple electrodes for biometric signal measurement.

The audio module 205 and 208 may include the microphone hole 205 and the speaker hole 208. A microphone for obtaining external sound may be disposed in the microphone hole 205, and in an embodiment of the disclosure, multiple microphones may be arranged therein to detect a direction of sound. The speaker hole 208 may be used as an external speaker and a call receiver. In an embodiment of the disclosure, a speaker may be included without a speaker hole (e.g., a piezo speaker).

The sensor module 265 may generate an electrical signal or a data value corresponding to an internal operational state or an external environmental state of the first wearable device 201. The sensor module 265, for example, the biometric sensor module 265 (e.g., heart rate monitor (HRM) sensor) disposed at the second surface 212 of the housing 210 may include an electrocardiogram (ECG) sensor 265a including at least two electrodes a1 and a2 for electrocardiogram measurement and a PPG sensor 265b for heart rate measurement. The first wearable device 201 may further include a sensor module that is not illustrated, for example at least one of a gesture sensor, a gyro sensor, an atmospheric pressure sensor, a magnetic sensor, an acceleration sensor, a grip sensor, a color sensor, an infrared (IR) sensor, a biometric sensor, a temperature sensor, a humidity sensor, or an illuminance sensor.

The key input devices 202, 203, and 204 may include the wheel key 202 disposed on the first surface 211 of the housing 210 and rotatable in at least one direction and/or the side key buttons 203 and 204 arranged on the lateral surface 213 of the housing 210. The wheel key 202 may have a shape corresponding to a shape of the front plate 222. In another embodiment of the disclosure, some of the key input devices 202, 203, and 204 may be implemented in a different type, such as a soft key on the display 220. The connector hole 209 may receive a connector (e.g., a USB connector) for transmission and reception of power and/or data with an external electronic device, and may include another connector hole (not illustrated) capable of receiving a connector for transmission and reception of an audio signal with an external electronic device. The first wearable device 201 may further include, for example, a connector cover (not illustrated) that covers at least a part of the connector hole 209 and blocks inflow of external foreign material through the connector hole.

The wearing members 250 and 260 may be detachably attached to at least a partial area of the housing 210 by using locking members 251 and 261. The locking members 251 and 261 may include engagement components, such as pogo pins, and the components may be replaced with protrusions or recesses formed on the locking members 251 and 261 according to an embodiment. For example, the wearing members 250 and 260 may be coupled to the housing 210 by being engaged with recesses or protrusions formed on the housing 210. The wearing members 250 and 260 may include one or more of a fixing member 252, a fixing member fastening hole 253, a band guide member 254, a band fixing ring 255.

The fixing member 252 may be configured to fix the housing 210 and the wearing members 250 and 260 to a part (e.g., wrist or ankle) of a user's body. The fixing member fastening hole 253 may correspond to the fixing member 252 and fix the housing 210 and the wearing members 250 and 260 to a part (e.g., wrist or ankle) of a user's body. The band guide member 254 may be configured to restrict a movement range of the fixing member 252 when the fixing member 252 is fastened to the fixing member fastening hole 253, thereby enabling the wearing members 250 and 260 to come in close contact with and be attached to a part of a user's body. The band fixing ring 255 may restrict a movement range of the wearing members 250 and 260 in a state where the fixing member 252 and the fixing member fastening hole 253 have been fastened.

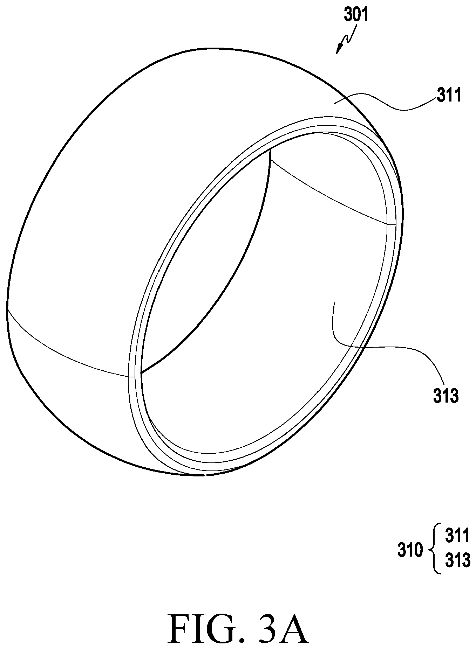

FIG. 3A is a perspective view illustrating a second wearable device 301 according to an embodiment of the disclosure.

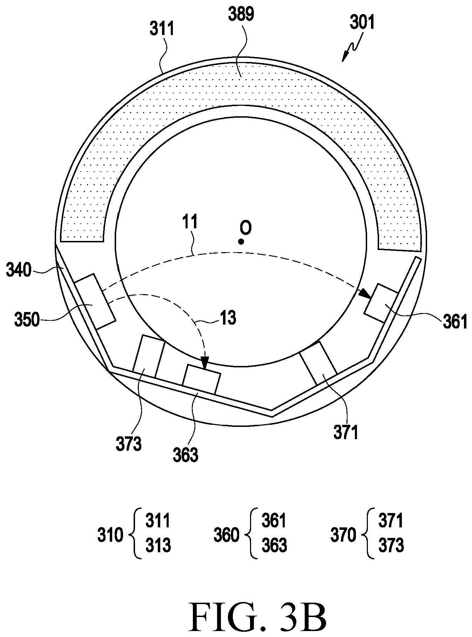

FIG. 3B is a sectional view of the second wearable device 301 according to an embodiment of the disclosure.

Referring to FIGS. 3A and 3B, the second wearable device 301 may include a housing 310. The housing 310 may configure the overall external appearance of the second wearable device 301.

According to an embodiment of the disclosure, the housing 310 may have an annular shape. The housing 310 may include an opening configured to accommodate a user's finger. For example, the opening may be defined as a hole formed through the housing 310.

According to an embodiment of the disclosure, the housing 310 may include an external housing part 311 or an internal housing part 313. The internal housing part 313 may be coupled to the external housing part 311. According to an embodiment of the disclosure, the external housing part 311 and the internal housing part 313 may be separately manufactured and combined or may be integrally configured.

According to an embodiment of the disclosure, the external housing part 311 may include a material capable of resisting external impact and/or scratches and implementing a design feature. For example, the external housing part 311 may include at least one of titanium, stainless steel, or ceramic. The external housing part 311 may be colored or coated for design implementation.

According to an embodiment of the disclosure, the internal housing part 313 may be a part coming into contact with a user's finger when the user puts on the second wearable device 301. The internal housing part 313 may be manufactured of a material, such as, a molding material, transparent plastic, or glass for sensing. For example, the internal housing part 313 may be configured to be at least partially transparent. For example, the internal housing part 313 may include a material capable of transmitting light for measuring biometric information. At least a part of the internal housing part 313 may be manufactured of a material substantially identical or similar to that of the external housing part 311. In addition, at least a part of the internal housing part 313 may include a metallic material for measuring biometric information.

According to an embodiment of the disclosure, the external housing part 311 and the internal housing part 313 may be coupled so that an inner space of the housing 310 is provided. In the inner space of the housing 310, various electrical/electronic components of the second wearable device 301 may be arranged and/or mounted. For example, the housing 310 may accommodate various electrical/electronic components.

According to an embodiment of the disclosure, the second wearable device 301 may include a circuit board 340, at least one light emitter 350, at least one sensor 360, at least one blocking member 370, or a battery 389 (e.g., the battery 189 in FIG. 1).

According to an embodiment of the disclosure, the circuit board 340 may be disposed in the inner space of the housing 310. The circuit board 340 may include at least one of a printed circuit board (PCB), a flexible printed circuit board (FPCB), or a rigid-flexible PCB (RF-PCB).

According to an embodiment of the disclosure, various electrical/electronic components may be arranged and/or mounted on the circuit board 340. For example, a processor (e.g., the processor 120 in FIG. 1), memory (e.g., the memory 130 in FIG. 1), a communication module (e.g., the communication module 190 in FIG. 1), or a sensor module (e.g., the sensor module 176 in FIG. 1, or the at least one light emitter 350 or the at least one sensor 360 in FIG. 3B) may be mounted on the circuit board 340.

According to an embodiment of the disclosure, the circuit board 340 may include multiple printed circuit boards. For example, the multiple printed circuit boards may be arranged according to a shape of the inner space of the housing 310 or may be electrically connected to each other. The circuit board 340 may include a flexible printed circuit board (FPCB). For example, the flexible printed circuit board may be at least partially bent according to the shape of the inner space of the housing 310.

According to an embodiment of the disclosure, the battery 389 may include a non-rechargeable primary battery, a rechargeable secondary battery, or a fuel cell as a device for supplying power to an element of the second wearable device 301. The battery 389 may be integrally disposed in the second wearable device 301, and may be detachably attached to the second wearable device 301. According to an embodiment of the disclosure, the battery 389 may be configured to be one integrated battery or include multiple separable batteries. The battery 389 may include a battery pack that is bent according to the shape of the inner space of the housing 310. The battery 389 may include multiple battery packs that are not bent by the housing 310. The battery 389 may include a bendable battery pack and non-bendable multiple battery packs.

According to an embodiment of the disclosure, the second wearable device 301 may include a power management module (e.g., the power management module 188 in FIG. 1) disposed on the circuit board 340.

According to an embodiment of the disclosure, the second wearable device 301 may include a sensor for obtaining (or measuring) at least one piece of biometric information. For example, the at least one piece of biometric information may include at least one of information on a user's blood pressure, information on a user's oxygen saturation, or information on a user's heart rate. For example, the sensor may include a photoplethysmography (PPG) sensor for measuring a blood pressure, oxygen saturation, or heart rate.

According to an embodiment of the disclosure, the PPG sensor may include a light source (e.g., the at least one light emitter 350) configured to emit light of two wavelength bands (e.g., RED wavelength band, or infrared wavelength band). The PPG sensor may include a light receiver (e.g., the at least one sensor 360) configured to detect at least a part of light reflected by or transmitted through a part (e.g., a finger or the skin or a blood vessel of a finger) of a user's body.

According to an embodiment of the disclosure, the at least one light emitter 350 may emit, for measurement of a user's oxygen saturation, respective light rays of substantially identical or different wavelengths to radiate the light rays to a part (e.g., a finger or the skin and/or a blood vessel of a finger) of the user's body. The light emitter 350 may be configured to emit light of multiple wavelength bands including a red wavelength and an infrared wavelength. For example, the at least one light emitter 350 may emit light of various bands and may include at least one of a light emitting diode (LED), a laser diode, or a vertical cavity surface emitting laser (VCSEL). The at least one light emitter 350 may be disposed and/or mounted on the circuit board 340. The at least one light emitter 350 may be configured to divide time to sequentially (or repeatedly) emit light of different wavelength bands. For example, the light emitter 350 may be configured to emit light through the internal housing part 313.

According to an embodiment of the disclosure, the at least one sensor 360 may accumulate photocharges corresponding to the amount of light incident after being reflected by or transmitted through a part of a user's body, and convert, into a digital signal, a biometric signal of an analog current type according to the accumulated photocharges. For example, light (or optical signal) obtained (or detected) through the at least one sensor 360 may be converted through an analog-to-digital converter (ADC) and then be stored in memory or a sensor buffer. The at least one sensor 360 may include at least one of a photodiode (PD), a photo transistor, a charge-coupled device (CCD), or a complementary metal oxide semiconductor (CMOS). The at least one sensor 360 is not limited thereto, and may include various devices capable of converting an incident optical signal to an electrical signal.

According to an embodiment of the disclosure, the at least one sensor 360 may include a first sensor 361 or a second sensor 363. The first sensor 361 and/or the second sensor 363 may be disposed and/or mounted on the circuit board 340.

According to an embodiment of the disclosure, the first sensor 361 may be disposed to be farther away from the at least one light emitter 350 than the second sensor 363. For example, the first sensor 361 may be positioned to be farther away from the at least one light emitter 350 than the second sensor 363 with respect to a circumferential direction of the housing 310. For example, the distance between the first sensor 361 and the light emitter 350 may be greater than that between the second sensor 363 and the light emitter 350.

According to an embodiment of the disclosure, the angle formed by the at least one light emitter 350 and the second sensor 363 with respect to a center O of the second wearable device 301 of the annular shape may be smaller than that formed by the at least one light emitter 350 and the first sensor 361 with respect to the center O of the second wearable device 301.

According to an embodiment of the disclosure, the first sensor 361 may be configured to receive light transmitted through a part of a user's body. The first sensor 361 may be referred as a transmissive sensor. The first sensor 361 may receive at least some of light transmitted through a part of a user's body, convert the transmitted light into an electrical signal, and transfer the electrical signal to a processor (e.g., the processor 120 in FIG. 1). According to an embodiment of the disclosure, the second sensor 363 may be configured to receive light reflected by a part of a user's body. The second sensor 363 may be referred as a reflective sensor. The second sensor 363 may receive at least some of light reflected by a part of a user's body, convert the reflected light into an electrical signal, and transfer the electrical signal to a processor (e.g., the processor 120 in FIG. 1).

According to an embodiment of the disclosure, light emitted from the at least one light emitter 350 may reach the first sensor 361 along a first optical path 11 or reach the second sensor 363 along a second optical path 13. For example, the first optical path 11 may be a path in which light is transmitted through a part (e.g., a finger, the skin of a finger, or a blood vessel of a finger) of a user's body, and the second optical path 13 may be a path in which light is reflected by a part of a user's body.

According to an embodiment of the disclosure, the second wearable device 301 may include the at least one blocking member 370. The at least one blocking member 370 may include a material that absorbs or blocks light. The at least one blocking member 370 may be configured to block light emitted from the at least one light emitter 350, from being propagated in the inner space of the housing 310.

According to an embodiment of the disclosure, the at least blocking member 370 may include a first wall 371 or a second wall 373. The first wall 371 may be positioned between the first sensor 361 and the second sensor 363 in the inner space of the housing 310. The second wall 373 may be positioned between the second sensor 363 and the at least one light emitter 350 in the inner space of the housing 310.

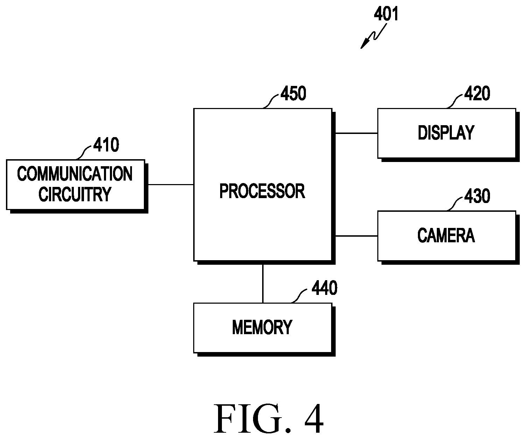

FIG. 4 is a block diagram of an electronic device 401 according to an embodiment of the disclosure.

Referring to FIG. 4, in an embodiment of the disclosure, an electronic device 401 may be the electronic device 101 of FIG. 1.

In an embodiment of the disclosure, the electronic device 401 may include communication circuitry 410, a display 420, a camera 430, memory 440, and/or a processor 450.

In an embodiment of the disclosure, the communication circuitry 410 may be the communication module 190 in FIG. 1. The communication circuitry 410 may support communication performed between the electronic device 401 and an external electronic device (e.g., the electronic device 102, or the electronic device 104). For example, the communication circuitry 410 (e.g., short-range communication circuit) may enable the electronic device 401 to be wirelessly connected to a first wearable device 501 and a second wearable device 601.

In an embodiment of the disclosure, the display 420 may be the display module 160 in FIG. 1.

In an embodiment of the disclosure, the display 420 may display various information. For example, the display 420 may display information required for performing an operation of measuring a blood pressure. For example, the display 420 may, based on a blood pressure being obtained, display the obtained blood pressure (e.g., a blood pressure value). However, information displayed by the display 420 is not limited to the above examples.

In an embodiment of the disclosure, the camera 430 may be the camera module 180 in FIG. 1.

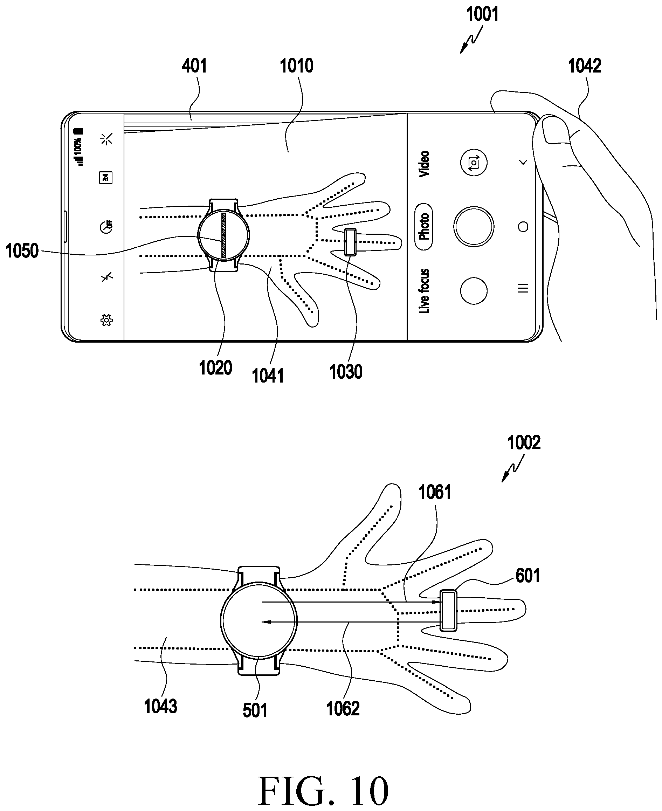

In an embodiment of the disclosure, the camera 430 may obtain (e.g., capture) an image for the first wearable device 501 and the second wearable device 601 worn on a user while the first wearable device 501 is displaying an object having a designated length (e.g., a bar-shaped image having an edge of about 1 cm as a real length). Based on the captured image, the distance between the first wearable device 501 and the second wearable device 601 may be obtained. An operation of, based on the captured image, obtaining the distance between the first wearable device 501 and the second wearable device 601 will be described later in detail with reference to FIG. 10.

In an embodiment of the disclosure, the memory 440 may be the memory 130 in FIG. 1.

In an embodiment of the disclosure, the memory 440 may store information required for performing an operation of measuring a blood pressure.

In an embodiment of the disclosure, the processor 450 may be the processor 120 in FIG. 1.

In an embodiment of the disclosure, the processor 450 may control the overall operation of measuring a blood pressure (e.g., a blood pressure value). The processor 450 may include one or more processors for performing an operation of measuring a blood pressure. An operation performed by the processor 450 to measure a blood pressure will be described in detail later.

FIG. 4 illustrates that the electronic device 401 includes the communication circuitry 410, the display 420, the camera 430, the memory 440, and/or the processor 450, but the disclosure is not limited thereto. For example, the electronic device 401 may further include at least one component included in the electronic device 101 in FIG. 1.

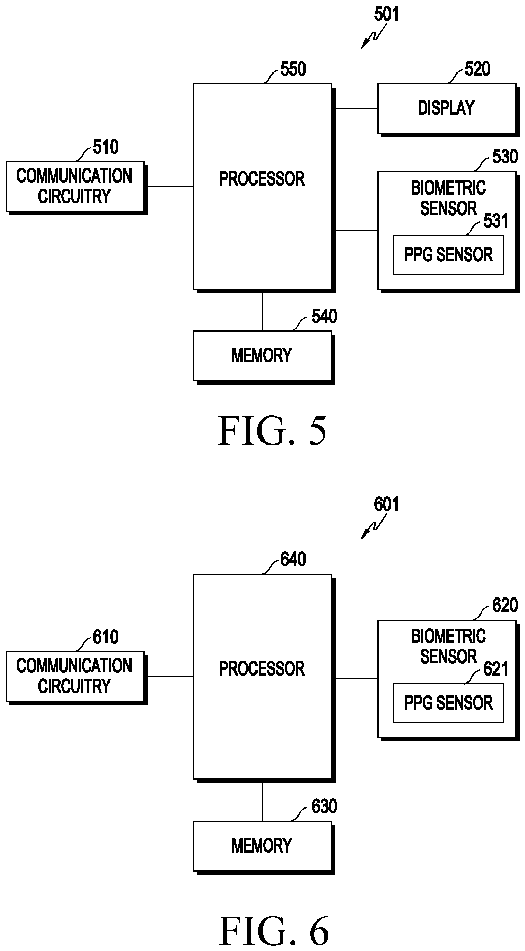

FIG. 5 is a block diagram of a first wearable device 501 according to an embodiment of the disclosure.

Referring to FIG. 5, in an embodiment of the disclosure, a first wearable device 501 may be the electronic device 101 of FIG. 1 or the first wearable device 201 in FIGS. 2A and 2B. For example, the first wearable device 501 may include a smart watch that performs an operation while being worn on a user's wrist.

In an embodiment of the disclosure, the first wearable device 501 may include communication circuitry 510, a display 520, a biometric sensor 530, memory 540, and/or a processor 550.

In an embodiment of the disclosure, the communication circuitry 510 may be the communication module 190 in FIG. 1. The communication circuitry 510 may support communication performed between the first wearable device 501 and an external electronic device (e.g., the electronic device 401 and/or a second wearable device 601).

In an embodiment of the disclosure, the display 520 may be the display module 160 in FIG. 1 or the display 220 in FIGS. 2A and 2B.

In an embodiment of the disclosure, the biometric sensor 530 may include at least one of sensors included in the sensor module 176 in FIG. 1 or sensors included in the sensor module 265 in FIGS. 2A and 2B.

In an embodiment of the disclosure, the biometric sensor 530 may include a PPG sensor 531. The PPG sensor (e.g., the PPG sensor 265b in FIGS. 2A and 2B) may obtain (e.g., measure) a PPG signal. However, a sensor included in the biometric sensor 530 is not limited to the PPG sensor 531. For example, the biometric sensor 530 may further include the ECG sensor 265a including the at least two electrodes a1 and a2 for electrocardiogram measurement, illustrated in FIG. 2B.

In an embodiment of the disclosure, the memory 540 may be the memory 130 in FIG. 1.

In an embodiment of the disclosure, the memory 540 may store information required for performing an operation of measuring a blood pressure.

In an embodiment of the disclosure, the processor 550 may be the processor 120 in FIG. 1.

In an embodiment of the disclosure, the processor 550 may perform a part of an operation for measuring a blood pressure. The processor 550 may include one or more processors for performing the part of the operation for measuring a blood pressure. The part of the operation performed by the processor 550 to measure a blood pressure will be described in detail later.

FIG. 5 illustrates that the first wearable device 501 includes the communication circuitry 510, the display 520, the biometric sensor 530, the memory 540, and/or the processor 550, but the disclosure is not limited thereto. For example, the first wearable device 501 may further include at least one component included in the electronic device 101 in FIG. 1 or the first wearable device 201 in FIGS. 2A and 2B.

FIG. 6 is a block diagram of a second wearable device 601 according to an embodiment of the disclosure.

Referring to FIG. 6, in an embodiment of the disclosure, a second wearable device 601 may be the electronic device 101 of FIG. 1 or the second wearable device 301 in FIGS. 3A and 3B. For example, the second wearable device 601 may include a smart ring that performs an operation while being worn on a user's finger.

In an embodiment of the disclosure, the second wearable device 601 may include communication circuitry 610, a biometric sensor 620, memory 630, and/or a processor 640.

In an embodiment of the disclosure, the communication circuitry 610 may be the communication module 190 in FIG. 1. The communication circuitry 610 may support communication performed between the second wearable device 601 and an external electronic device (e.g., the electronic device 401 and/or the first wearable device 501).

In an embodiment of the disclosure, the biometric sensor 620 may include at least one of sensors included in the sensor module 176 in FIG. 1.

In an embodiment of the disclosure, the biometric sensor 620 may include a PPG sensor 621.

In an embodiment of the disclosure, the PPG sensor 621 may obtain (e.g., measure) a PPG signal. The PPG sensor 621 may include the at least one light emitter 350 and the at least one sensor 360 in FIG. 3B to obtain a PPG signal.

However, a sensor included in the biometric sensor 620 is not limited to the PPG sensor 621.

In an embodiment of the disclosure, the memory 630 may be the memory 130 in FIG. 1.

In an embodiment of the disclosure, the memory 630 may store information required for performing an operation of providing a blood pressure.

In an embodiment of the disclosure, the processor 640 may be the processor 120 in FIG. 1.

In an embodiment of the disclosure, the processor 640 may perform a part of an operation for measuring a blood pressure. The processor 640 may include one or more processors for performing the part of the operation for measuring a blood pressure. The part of the operation performed by the processor 550 to measure a blood pressure will be described in detail later.

FIG. 6 illustrates that the second wearable device 601 includes the communication circuitry 610, the biometric sensor 612, the memory 630, and/or the processor 640, but the disclosure is not limited thereto. For example, the second wearable device 601 may further include at least one component included in the electronic device 101 in FIG. 1 or the second wearable device 301 in FIGS. 3A and 3B.

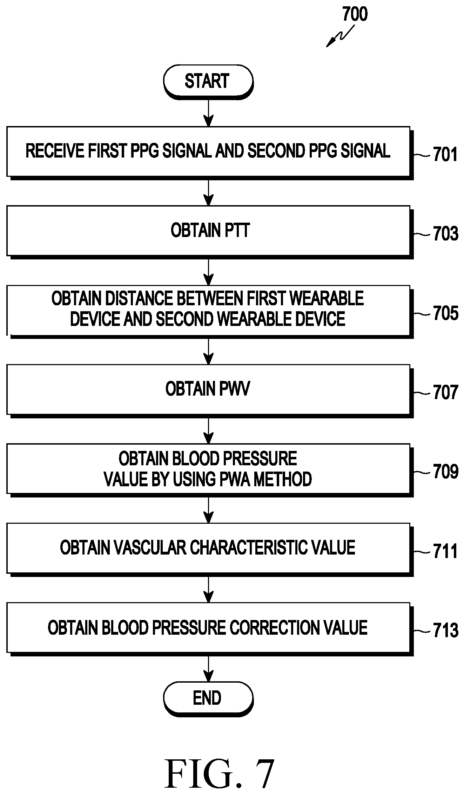

FIG. 7 is a flowchart 700 illustrating a method of measuring a blood pressure (e.g., a blood pressure value) according to an embodiment of the disclosure.

Referring to FIG. 7, in operation 701, in an embodiment of the disclosure, the processor 450 may receive, through the communication circuitry 410, a first PPG signal (hereinafter, a PPG signal received from the first wearable device 501 is referred as a "first PPG signal") from the first wearable device 501 and receive a second PPG signal (hereinafter, a PPG signal received from the second wearable device 601 is referred as a "second PPG signal") from the second wearable device 601.

In an embodiment of the disclosure, the processor 450 may receive, from the first wearable device 501 through the communication circuitry 410, a first PPG signal obtained (e.g., measured) by the first wearable device 501 and time information (hereinafter, this is referred to as "first time information") on a time at which the first PPG signal is obtained. For example, the processor 450 may receive, from the first wearable device 501 through the communication circuitry 410, values of a first PPG signal measured by the first wearable device 501 by using a PPG sensor and respective time points at which the values of the first PPG signal are measured.

In an embodiment of the disclosure, the processor 450 may receive, from the second wearable device 601 through the communication circuitry 410, a second PPG signal obtained by the second wearable device 601 and time information (hereinafter, this is referred to as "second time information") on a time at which the second PPG signal is obtained. For example, the processor 450 may receive, from the second wearable device 601 through the communication circuitry 410, values of a second PPG signal measured by the second wearable device 601 by using a PPG sensor and respective time points at which the values of the second PPG signal are measured.

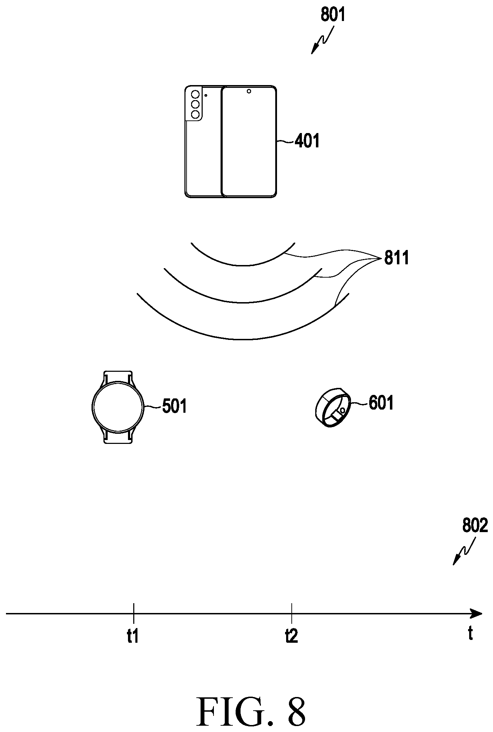

In an embodiment of the disclosure, first time information on a time at which a first PPG signal is obtained and second time information on a time at which a second PPG signal is obtained may be synchronized time information. For example, first time information and second time information may be time information on times at which PPG signals are obtained by the first wearable device 501 and the second wearable device 601 for which times serving as a reference (hereinafter, these are referred as "reference times") are configured to be identical. Hereinafter, an operation in which the first wearable device 501 and the second wearable device 601 configure reference times to be identical may be referred as an operation of synchronizing the first wearable device 501 and the second wearable device 601. The operation of synchronizing the first wearable device 501 and the second wearable device 601 will be described with reference to FIG. 8.

FIG. 8 is a diagram illustrating a method of synchronizing a first wearable device 501 and a second wearable device 601 according to an embodiment of the disclosure.

Referring to FIG. 8, in an embodiment of the disclosure, as indicated by reference numeral 801, the processor 450 may transmit (e.g., broadcast) an advertising signal 811 (e.g., BLE beacon signal) including time information (e.g., time stamp of the electronic device 401) by using the communication circuitry 410 (e.g., Bluetooth^{™} communication circuit). For example, as indicated by reference numeral 802, the processor 450 may periodically transmit an advertising packet including time information of the electronic device 401 (e.g., local operating system (OS) time information of the electronic device 401) at a first time point t1 by using a Bluetooth^{™} (e.g., Bluetooth^{™} low energy (BLE) or Bluetooth^{™} classic) communication circuit.

In an embodiment of the disclosure, the first wearable device 501 and the second wearable device 601 may receive the signal 811 including time information of the electronic device 401 transmitted from the electronic device 401, at a substantially identical time. For example, as indicated by reference numeral 802, the first wearable device 501 and the second wearable device 601 may substantially simultaneously receive, at a second time point t2, the signal 811 having been transmitted from the electronic device 401 at the first time point t1.

In an embodiment of the disclosure, the first wearable device 501 and the second wearable device 601 may be synchronized by configuring, as reference times, the second time point t2 at which the first wearable device 501 and the second wearable device 601 have received the signal 811 substantially simultaneously.

However, the method of synchronizing the first wearable device 501 and the second wearable device 601 is not limited to the above example. For example, in a case where the electronic device 401, the first wearable device 501, and the second wearable device 601 support short-range communication (e.g., Wi-Fi) other than Bluetooth^{™}, the electronic device 401, the first wearable device 501, and the second wearable device 601 may perform an operation of synchronizing the first wearable device 501 and the second wearable device 601, by using the different short-range communication. For example, the electronic device 401, the first wearable device 501, and the second wearable device 601 may perform an operation of synchronizing the first wearable device 501 and the second wearable device 601, by using an audio signal using an inaudible frequency.

Referring to FIG. 7 again, in an embodiment of the disclosure, after the first wearable device 501 and the second wearable device 601 are synchronized, the first wearable device 501 and the second wearable device 601 may obtain PPG signals through the PPG sensors 531 and 621. For example, each of the first wearable device 501 and the second wearable device 601 may perform an operation of obtaining PPG signals, according to a pre-configured period. For example, each of the first wearable device 501 and the second wearable device 601 may perform an operation of obtaining PPG signals, at the time of a user input. For example, each of the first wearable device 501 and the second wearable device 601 may perform an operation of obtaining PPG signals, when a signal including a command to obtain a PPG signal is received from the electronic device 401.

In an embodiment of the disclosure, after the first wearable device 501 and the second wearable device 601 are synchronized, each of the first wearable device 501 and the second wearable device 601 may perform an operation of obtaining a PPG signal, when a designated condition is satisfied. For example, the first wearable device 501 (and the second wearable device 601) may perform an operation of obtaining a PPG signal, based on the size of movement of the first wearable device 501 being equal to or smaller than a threshold size. For example, the first wearable device 501 (and the second wearable device 601) may perform an operation of obtaining a PPG signal through the PPG sensor 531, based on the size of movement of the first wearable device 501 being equal to or smaller than a threshold size and a heart rate obtained through the first wearable device 501 being equal to or smaller than a threshold (and/or based on a heart rate variability).

In an embodiment of the disclosure, each of the first wearable device 501 and the second wearable device 601 may perform an operation of obtaining a first PPG signal and a second PPG signal for a pre-configured time (e.g., about 1 minute).

In an embodiment of the disclosure, the first wearable device 501 may include a smart watch that performs an operation of obtaining a first PPG signal, while being worn on a user's first position (e.g., the user's wrist). The second wearable device 601 may include a smart ring that performs an operation of obtaining a second PPG signal, while being worn on a user's second position (e.g., the user's finger).

In an embodiment of the disclosure, the first wearable device 501 and the second wearable device 601 may obtain a first PPG signal and a second PPG signal and then transmit the obtained first PPG signal and second PPG signal to the electronic device 401. The processor 450 may receive a first PPG signal and a second PPG signal from the first wearable device 501 and the second wearable device 601 through the communication circuitry 410 to obtain the first PPG signal and the second PPG signal.

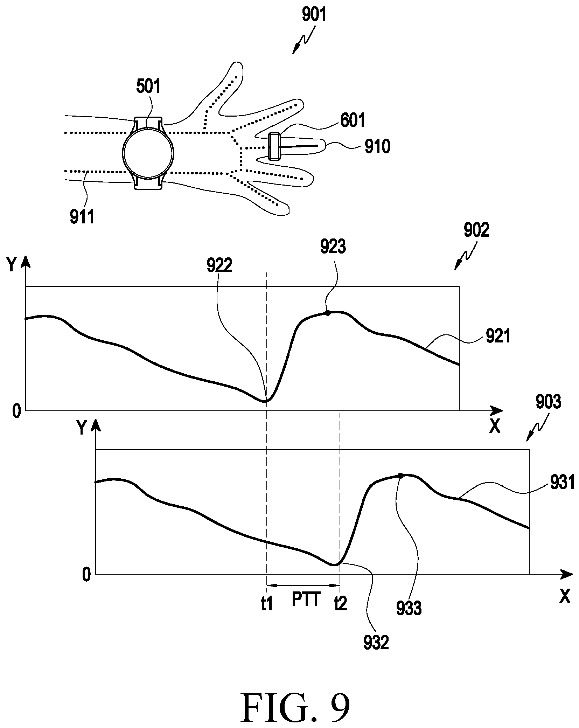

In operation 703, in an embodiment of the disclosure, the processor 450 may obtain, based on the first PPG signal and the second PPG signal, a pulse transit time (PTT) during which a pulse wave is transferred from the user's first position (hereinafter, this is also referred to as a "first position") on which the first wearable device 501 is worn, to the user's second position (hereinafter, this is also referred to as a "second position") on which the second wearable device 601 is worn. Hereinafter, an operation of obtaining a PTT, based on a first PPG signal and a second PPG signal is described with reference to FIG. 9.

FIG. 9 is a diagram illustrating a method of obtaining a PTT, based on a first PPG signal and a second PPG signal according to an embodiment of the disclosure.

Referring to FIG. 9, in an embodiment of the disclosure, reference numeral 901 may indicate the first wearable device 501 and the second wearable device 601 worn on a user 910. For example, the first wearable device 501 may be worn on a wrist of the user 910 and the second wearable device 601 may be worn on a finger of the user 910. Reference numeral 901 to reference numeral 911 may show a blood vessel (e.g., artery) of the user 910.

In an embodiment of the disclosure, reference numeral 902 may indicate a graph including a first PPG signal 921 (e.g., the wave form of the first PPG signal), and reference numeral 903 may indicate a graph including a second PPG signal 931 (e.g., the wave form of the second PPG signal). In each of the graphs of reference numeral 902 and reference numeral 903, the x axis represents time and the y axis represents the size (e.g., amplitude) of the PPG signal.

In an embodiment of the disclosure, in each of the graphs of reference numeral 902 and reference numeral 903, the time represented by the x axis may be time having a reference time (e.g., the second time point t2) as a reference. In each of the graphs of reference numeral 902 and reference numeral 903, the y axis may represent the size of a PPG signal measured by a wearable device (e.g., each of the first wearable device 501 and the second wearable device 601) by using a reference time (e.g., the second time point t2) as a reference.

In an embodiment of the disclosure, the processor 450 may obtain (e.g., extract), from each of the first PPG signal 921 and the second PPG signal 931, one or more feature points including a point having a maximum size, a point having a minimum size, an inflection point, and/or a point having a maximum slope. However, the one or more feature points are not limited to the above examples.

In an embodiment of the disclosure, the processor 450 may obtain a PTT by comparing the times of mutually corresponding feature points in the first PPG signal 921 and the second PPG signal 931 (e.g., based on the difference between the times of mutually corresponding feature points). For example, the processor 450 may identify times t1 and t2 of points 922 and 932 corresponding to each other and having minimum sizes in the first PPG signal 921 and the second PPG signal 931. The processor 450 may subtract the time t1 corresponding to the point 922 from the time t2 corresponding to the point 932 to obtain (e.g., calculate) a PTT. In the above example, obtaining a PTT, based on the times t1 and t2 corresponding to the points 922 and 932 having minimum sizes is described, but the disclosure is not limited thereto. For example, the processor 450 may obtain a PTT by subtracting a time corresponding to a point 923 having a maximum size in the first PPG signal 921 from a time corresponding to a point 933 having a maximum size in the second PPG signal 931.

Referring to FIG. 7 again, in operation 705, in an embodiment of the disclosure, the processor 450 may obtain the distance between the first wearable device 501 and the second wearable device 601.

In an embodiment of the disclosure, the distance between the first wearable device 501 and the second wearable device 601 may be substantially identical to the distance between the user's first position (e.g., a position on which the first PPG signal is measured) on which the first wearable device 501 is worn and the user's second position (e.g., a position on which the second PPG signal is measured) on which the second wearable device 601 is worn. Hereinafter, an operation of obtaining the distance between the first wearable device 501 and the second wearable device 601 is described with reference to FIG. 10.

FIG. 10 is a diagram illustrating a method of obtaining a distance between a first wearable device 501 and a second wearable device 601 according to an embodiment of the disclosure.

Referring to FIG. 10, in an embodiment of the disclosure, the processor 450 may obtain (e.g., calculate) the distance between the first wearable device 501 and the second wearable device 601, based on an image for the first wearable device 501 and the second wearable device 601 worn on a user.

In an embodiment of the disclosure, the processor 450 may transmit, to the first wearable device 501 through the communication circuitry 410, a signal that causes the first wearable device 501 to display, through the display 520, an object having a designated length (e.g., a bar-shaped image having an edge of about 1 cm as a real length). For example, the processor 450 may, based on the electronic device 401 operating in a mode for obtaining the distance between the first wearable device 501 and the second wearable device 601, control the first wearable device 501 such that the display 520 of the first wearable device 501 displays an object having a designated length.

In an embodiment of the disclosure, while the first wearable device 501 is displaying the object, the processor 450 may obtain an image for the first wearable device 501 and the second wearable device 601 worn on the user by using the camera 430. For example, as indicated by reference numeral 1001, in a state where the user is holding the electronic device 401 by using the user's right hand 1042, the processor 450 may obtain an image for the first wearable device 501 and the second wearable device 601 worn on the left hand by using the camera 430.

In an embodiment of the disclosure, as indicated by reference numeral 1001, the processor 450 may display, through the display 420, an image 1010 which is obtained through the camera 430 and includes a part 1020 representing the first wearable device 501, a part 1030 representing the second wearable device 601, and a part 1041 representing the left hand.

In an embodiment of the disclosure, the processor 450 may obtain (e.g., identify), in the image 1010, the length (hereinafter, this is referred to as a "first length") of a part 1050 representing an object that has a designated length and is displayed through the first wearable device 501 (e.g., a bar-shaped image having an edge of about 1 cm as a real length).

In an embodiment of the disclosure, the processor 450 may obtain (e.g., identify), from the image 1010, the distance (hereinafter, this is referred as a "first distance") between the part 1020 representing the first wearable device 501 and the part 1030 representing the second wearable device 601. For example, the processor 450 may identify, in the image 1010, the distance between the center of the part 1020 representing the first wearable device 501 and the center of the part 1030 representing the second wearable device 601.

In an embodiment of the disclosure, the processor 450 may obtain the distance between the first wearable device 501 and the second wearable device 601 (e.g., the real distance between the first wearable device 501 and the second wearable device 601), based on the designated length of the object, the first length, and the first distance. For example, the processor 450 may calculate the distance between the first wearable device 501 and the second wearable device 601 by using [Equation 1] below.$D = \left(first distance\right) / \left(first length\right) * \left(designated length of object\right)$

In an embodiment of the disclosure, in [Equation 1], D may denote the real distance between the first wearable device 501 and the second wearable device 601. In [Equation 1], the designated length of the object may indicate the real length (e.g., about 1 cm) of the object displayed through the display 520 of the first wearable device 501.

Although not illustrated in FIG. 10, in an embodiment of the disclosure, the processor 450 may obtain the distance between the first wearable device 501 and the second wearable device 601, based on an image for the first wearable device 501 and the second wearable device 601 worn on the user, and a real length of the display 520 of the first wearable device 501 (e.g., a real diameter of the display 520 of the first wearable device 501) replaceable with the designated length of the object described above.

In an embodiment of the disclosure, the processor 450 may receive identification information of the first wearable device 501 (e.g., a model name of the first wearable device 501) from the first wearable device 501 through the communication circuitry 410, or may obtain identification information of the first wearable device 501 stored in the memory 440 from the memory 440. The processor 450 may transmit identification information of the first wearable device 501 to a server through the communication circuitry 410 to receive the real length (hereinafter, this is referred to as a "second length") of the display 520 of the first wearable device 501 from the server. However, the disclosure is not limited thereto. For example, in a case where a second length is stored in the memory 440 as the real length of the display 520 of the first wearable device 501, the processor 450 may obtain the second length from the memory 440.

In an embodiment of the disclosure, the processor 450 may obtain a length (hereinafter, this is referred to as a "third length") of a part representing the display 520 of the first wearable device 501 in and based on an image, obtained through the camera 430, for the first wearable device 501 and the second wearable device 601 worn on the user. For example, when the part representing the display 520 of the first wearable device 501 in the image is displayed using a circular shape, the third length may be the length of the diameter of the circle. When the part representing the display 520 of the first wearable device 501 in the image is displayed using an oval shape, the third length may be the length between points at which the apsidal line of the oval and the oval cross over each other.

In an embodiment of the disclosure, the processor 450 may obtain (e.g., identify), from the image, a first distance between a part representing the first wearable device 501 and a part representing the second wearable device 601.

In an embodiment of the disclosure, the processor 450 may obtain the distance between the first wearable device 501 and the second wearable device 601, based on the first distance, the second length, and the third length. For example, the processor 450 may calculate the distance between the first wearable device 501 and the second wearable device 601 by using [Equation 2] below.$D = \left(first distance\right) / \left(third length\right) * \left(second length\right)$

In an embodiment of the disclosure, in [Equation 2], D may denote the real distance between the first wearable device 501 and the second wearable device 601.

In the above examples, the display 520 of the first wearable device 501 displays an object having a designated length or the real length of the display 520 of the first wearable device 501 is used, but the disclosure is not limited thereto. For example, in a case where the second wearable device 601 includes a display, the above operations may be performed using an object having a designated length displayed through the display of the second wearable device 601 or the real length of the display of the second wearable device 601.

In an embodiment of the disclosure, the processor 450 may obtain the distance between the first wearable device 501 and the second wearable device 601, based on a transmission/reception time of an audio signal (e.g., inaudible sound wave) between the first wearable device 501 and the second wearable device 601 and a velocity of the audio signal. For example, as indicated by reference numeral 1002, in a state where the first wearable device 501 and the second wearable device 601 are worn on a user 1043, the first wearable device 501 may transmit an audio signal to the second wearable device 601 in a direction indicated by an arrow 1061. The second wearable device 601 may transmit an audio signal to the first wearable device 501 in a direction indicated by an arrow 1062 in response to receiving the audio signal. The processor 450 may control an operation of transmitting and receiving an audio signal between the first wearable device 501 and the second wearable device 601. The processor 450 may receive, from the first wearable device 501 through the communication circuitry 410, a time from a time point at which the first wearable device 501 has transmitted the audio signal to the second wearable device 601 to a time point at which the first wearable device has received the audio signal from the second wearable device 601.

In an embodiment of the disclosure, the processor 450 may obtain the distance between the first wearable device 501 and the second wearable device 601, based on the received time and a velocity of the audio signal (e.g., the velocity of sound). For example, the processor 450 may obtain the real distance between the first wearable device 501 and the second wearable device 601, by multiplying the received time by the velocity of the audio signal and dividing the resultant value by 2.

In the above example, the real distance between the first wearable device 501 and the second wearable device 601 is obtained based on the time from a time point at which the first wearable device 501 has transmitted the audio signal to the second wearable device 601 to a time point at which the first wearable device has received the audio signal from the second wearable device 601. However, the disclosure is not limited thereto. For example, the processor 450 may obtain the distance between the first wearable device 501 and the second wearable device 601 by multiplying, by the velocity of an audio signal, a time from a time point at which the first wearable device 501 has transmitted the audio signal to a time point at which the second wearable device 601 has received the audio signal. For example, the processor 450 may obtain the distance between the first wearable device 501 and the second wearable device 601 by multiplying, by the velocity of an audio signal, a time from a time point at which the second wearable device 601 has transmitted the audio signal to a time point at which the first wearable device 501 has received the audio signal.

Referring to FIG. 7 again, in operation 707, in an embodiment of the disclosure, the processor 450 may obtain a pulse wave velocity (PWV) at which a pulse wave is transferred, based on the PTT obtained through operation 703 and the distance between the first wearable device 501 and the second wearable device 601 obtained through operation 705. For example, the processor 450 may obtain (e.g., calculate) a PWV by dividing, by the PTT, the distance between the first wearable device 501 and the second wearable device 601.

In operation 709, in an embodiment of the disclosure, the processor 450 may obtain a blood pressure by using a pulse wave analysis (PWA) method, based on the first PPG signal and/or the second PPG signal.

In an embodiment of the disclosure, the processor 450 may, as a pulse wave analysis (PWA) method, analyze the wave form of the first PPG signal and/or the wave form of the second PPG signal received through operation 701 to obtain (e.g., estimate) a blood pressure (e.g., hereinafter, a blood pressure obtained using the PWA method is referred as a "first blood pressure" or "P_{PWA}"). For example, the processor 450 may obtain a first blood pressure in the wave form of the first PPG signal (and/or the wave form of the second PPG signal), based on the size (e.g., amplitude) of one or more feature points (e.g., a point having a maximum size, a point having a minimum size, an inflection point, and/or a point having a maximum slope in the wave form of the first PPG signal), the time interval between one or more feature points, and/or the slope of one or more feature points in time. For example, the processor 450 may differentiate twice the first PPG signal (and/or second PPG signal) to obtain the wave form of an accelerated pulse wave (plethysmograph), and analyze a peak characteristic in the wave form of the accelerated pulse wave (e.g., each peak value and the time difference between peaks in the wave form of the accelerated pulse wave). Based on the peak characteristic, the processor 450 may obtain the first blood pressure by using a correlation, stored in the memory 440, between the first blood pressure (e.g., the value of the first blood pressure) and the peak characteristic of the wave form of the accelerated pulse wave. However, a method of obtaining a first blood pressure by using a PWA method is not limited to the above examples.

In an embodiment of the disclosure, the processor 450 may map the PWV obtained through operation 707 and the first blood pressure obtained through operation 709, and store the mapped PWV and first blood pressure in the memory 440.

In an embodiment of the disclosure, the processor 450 may perform operation 701 to operation 709 during a designated period (e.g., one day or one week). For example, the processor 450 may perform operation 701 to operation 709 during a designated period, to collect (e.g., store in the memory 440) multiple PWVs and multiple first blood pressures corresponding to (e.g., mapped to) the multiple PWVs, respectively.

In operation 711, in an embodiment of the disclosure, the processor 450 may obtain a value (or coefficient) related to a characteristic of a blood vessel (hereinafter, the value is referred as a "vascular characteristic value"), based on the PWV and the first blood pressure.

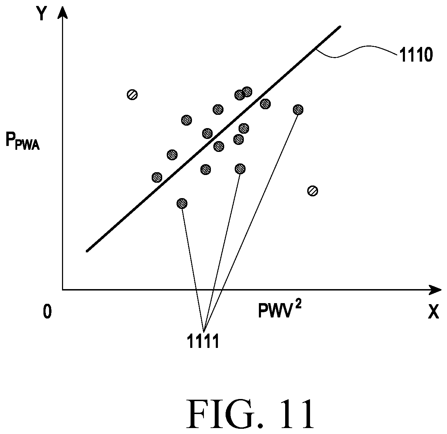

In an embodiment of the disclosure, the processor 450 may obtain a vascular characteristic value by using a PWV blood pressure model and a PWA blood pressure model. Hereinafter, an operation of obtaining a vascular characteristic value by using a PWV blood pressure model and a PWA blood pressure model is described with reference to FIG. 11.

FIG. 11 is a diagram illustrating a method of obtaining a vascular characteristic value by using a PWV blood pressure model and a PWA blood pressure model according to an embodiment of the disclosure.

Referring to FIG. 11, in an embodiment of the disclosure, [Equation 3] below may show a Fung's hyperelastic model as a PWV blood pressure model (e.g., a blood pressure model using PWV).$ln \left(\frac{P}{C}\right) + ln \left(\sqrt{1 + \frac{8 ρ}{{a}_{1}} \frac{{PWV}^{2}}{P}}-1\right) = \frac{{a}_{1}}{16} {\left(\sqrt{1 + \frac{8 ρ}{{a}_{1}} \frac{{PWV}^{2}}{P}}-1\right)}^{2}$

In an embodiment of the disclosure, in [Equation 3], P may denote a blood pressure. In [Equation 3], ρ indicates vascular density, and C and a₁ may be parameters related to vascular elasticity.

In an embodiment of the disclosure, [Equation 3] may be expressed as [Equation 4] below. For example, [Equation 3] may be approximated by [Equation 4] below.$P = α * \left({PWV}^{2}\right) + β$

In an embodiment of the disclosure, in [Equation 4], P may denote a blood pressure. In [Equation 4], α and β may be coefficients related to a vascular characteristic. For example, α and β may be coefficients related to, as a vascular characteristic, vascular elasticity, vascular radius, vascular thickness, and/or vascular density. In an embodiment of the disclosure, at least one of α, β, and γ described later may be included in a vascular characteristic value.

In the above example, a Fung's hyperelastic model is used as an example of a PWV blood pressure model, but the disclosure is not limited thereto, and a different PWV blood pressure model (e.g., MK & Hughes model) may be used.

In an embodiment of the disclosure, [Equation 5] below may indicate a pulse wave analysis (PWA) blood pressure model (e.g., a blood pressure model using PWA).$P = {P}_{PWA} + {P}_{cal}$

In an embodiment of the disclosure, in [Equation 5], P may denote a blood pressure. In [Equation 5], P_{PWA} denotes a blood pressure measured using a PWA method, and P_{cal} may denote a value for blood pressure correction (hereinafter, the value is referred as a "blood pressure correction value") for calculating a blood pressure (P) by correcting P_{PWA}.

In an embodiment of the disclosure, [Equation 6] and [Equation 7] below may be derived based on [Equation 4] and [Equation 5]. ${P}_{PWA} = α * \left({PWV}^{2}\right) + γ$$γ = β - {P}_{cal}$

In an embodiment of the disclosure, the processor 450 may obtain (e.g., calculate) α and γ as vascular characteristic values by using regression analysis, based on multiple PWVs and multiple first blood pressures (P_{PWA} values) (multiple first blood pressures corresponding to the multiple PWVs, respectively) (hereinafter, a PWV and a P_{PWA} value corresponding to the PWV are referred as a "PWV-and-P_{PWA} pair") obtained (e.g., collected) for a designated period (e.g., one day or one week). Hereinafter, α is referred as a "first vascular characteristic value", and γ may be referred as a "second vascular characteristic value". In addition, β may be referred as a "third vascular characteristic value".

In an embodiment of the disclosure, in [Equation 4], [Equation 5], [Equation 6], and [Equation 7], α (first vascular characteristic value), γ (second vascular characteristic value), β (third vascular characteristic value), and P_{cal} (blood pressure correction value) may be variable values. Hereinafter, an operation of obtaining (e.g., calculating) α, γ, β, and/or P_{cal} may include an operation of obtaining constant values of α, γ, β, and/or P_{cal}.

In an embodiment of the disclosure, the processor 450 may perform a regression analysis, based on identifying that the number of PWV-and-P_{PWA} pairs obtained for a designated period is equal to or greater than a designated number. The processor 450 may perform the regression analysis, thereby calculating an equation (hereinafter, this is also referred as a "relation formula") representing a correlation between PWV² and P_{PWA}. The processor 450 may obtain α and γ (e.g., α and γ as constant values) from the calculated equation.

In an embodiment of the disclosure, in FIG. 11, the X axis denotes PWV² and the Y axis may denote P_{PWA}. In FIG. 11, points 1111 may correspond to PWV-and-P_{PWA} pairs obtained during a designated period, respectively. For example, each of the points 1111 may be a point expressing a PWV-and-P_{PWA} pair by using a relation between PWV² and P_{PWA}.

In an embodiment of the disclosure, the processor 450 may perform a regression analysis (e.g., linear regression analysis) for the points 1111, thereby obtaining (e.g., calculate) a linear line 1110. For example, the processor 450 may perform a linear regression analysis for the points 1111, thereby calculating the linear line 1110 representing a relation formula between PWV² and P_{PWA}. The processor 450 may obtain (e.g., calculate) α and γ in [Equation 6] as vascular characteristic values, based on the linear line 1110 representing the relation formula between PWV² and P_{PWA}.

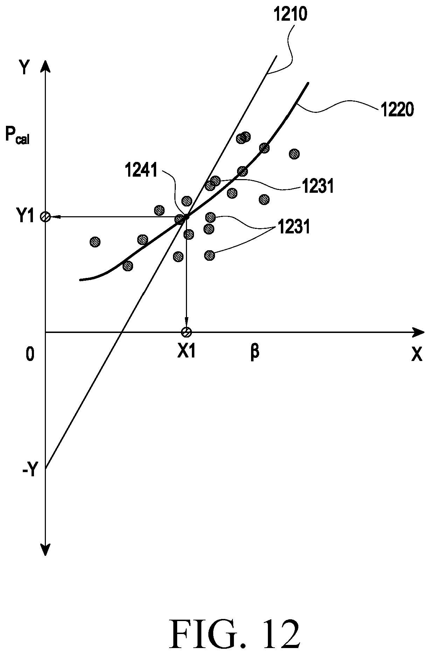

Referring to FIG. 7 again, in operation 713, in an embodiment of the disclosure, the processor 450 may obtain, based on the vascular characteristic value, a blood pressure correction value for correcting a second blood pressure to be obtained (e.g., P_{PWA} obtained at the time of next blood pressure measurement) (hereinafter, this is referred as a "blood pressure correction value") (e.g., P_{cal} in [Equation 5] and [Equation 7]. Hereinafter, an operation of obtaining a blood pressure correction value, based on a vascular characteristic value is described with reference to FIG. 12.

FIG. 12 is a diagram illustrating a method of obtaining a blood pressure correction value, based on a vascular characteristic value according to an embodiment of the disclosure.

Referring to FIG. 12, in an embodiment of the disclosure, the processor 450 may obtain a blood pressure correction value, based on a vascular characteristic value and a correlation between the vascular characteristic value and the blood pressure correction value. For example, the processor 450 may obtain a blood pressure correction value by using a correlation between a vascular characteristic value and the blood pressure correction value, based on the vascular characteristic value.

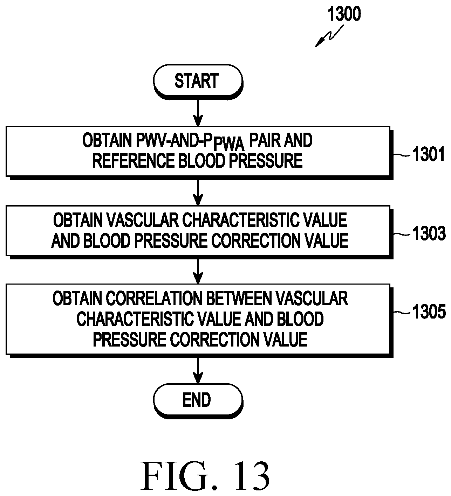

In an embodiment of the disclosure, the correlation between a vascular characteristic value and a blood pressure correction value may be a relation between β and P_{cal} in [Equation 7] (e.g., a function representing the relation between β and P_{cal}). In an embodiment of the disclosure, the correlation between a vascular characteristic value and a blood pressure correction value may be obtained in advance (e.g., before operation 701 is performed), based on data related to a blood vessel collected from multiple users (e.g., multiple electronic devices corresponding to the multiple users, respectively). An operation of obtaining the correlation between a vascular characteristic value and a blood pressure correction value will be described later with reference to FIG. 13.

In an embodiment of the disclosure, in FIG. 12, the X axis denotes β and the Y axis may denote P_{cal}. In FIG. 12, respective points 1231 may be points expressing, by using β and P_{cal}, respective data related to a blood vessel collected from multiple users (e.g., multiple electronic devices corresponding to the multiple users, respectively). In FIG. 12, a line 1220 may represent a function representing the relation between β and P_{cal} as a correlation between a vascular characteristic value and a blood pressure correction value.

In an embodiment of the disclosure, in FIG. 12, a line 1210 may be a linear line (e.g., primary line) representing the relation between β and P_{cal} and obtained by inputting γ obtained through operation 711 into [Equation 7]. For example, the y-intercept of a linear equation represented by the line 1210 is -y, and the slope thereof may be 1.

In an embodiment of the disclosure, the processor 450 may identify an intersection 1241 between the line 1210 and the line 1220. The processor 450 may obtain a Y-axis value (Y1) of the intersection 1241 as a blood pressure correction value (P_{cal}) (e.g., a blood pressure correction value (P_{cal}) as a constant value). However, the disclosure is not limited thereto. For example, the processor 450 may determine an X-axis value (X1) of the intersection 1241 as β in [Equation 7], and input the determined β and γ obtained through operation 711 into [Equation 7] to obtain a blood pressure correction value (P_{cal}).

In an embodiment of the disclosure, the processor 450 may perform an operation of obtaining a blood pressure correction value (P_{cal}) according to a designated period (e.g., one week, one month, or three months) to update the blood pressure correction value (P_{cal}).

Referring to FIG. 7 again, in an embodiment of the disclosure, after a blood pressure correction value (P_{cal}) is obtained through operation 713, the processor 450 may obtain a blood pressure (e.g., a third blood pressure to be provided to the user) by using the blood pressure correction value (P_{cal}). For example, after a blood pressure correction value (P_{cal}) is obtained, the processor 450 may perform an operation substantially identical to an operation described through operation 701 to receive, through the communication circuitry, a first PPG signal and/or a second PPG signal from the first wearable device 501 and/or the second wearable device 601. The processor 450 may perform an operation substantially identical to an operation described through operation 709 to obtain a first blood pressure (P_{PWA}) by using a PWA method, based on the first PPG signal and/or the second PPG signal. The processor 450 may obtain a second blood pressure to be provided to the user, based on the first blood pressure (P_{PWA}) and the blood pressure correction value (P_{cal}) (e.g., by adding the first blood pressure (P_{PWA}) and the blood pressure correction value (P_{cal})).

However, a method of obtaining a second blood pressure is not limited to the above example. In an embodiment of the disclosure, the processor 450 may perform the above operations to obtain α and β as vascular characteristic values related to a blood vessel. After α and β are obtained, the processor 450 may perform operation 701 to operation 707 to obtain a PWV. The processor 450 may input α, β, and the PWV into [Equation 4] to obtain a second blood pressure to be provided to the user.

FIG. 13 is a flowchart 1300 illustrating a method of obtaining a correlation between a vascular characteristic value and a blood pressure correction value according to an embodiment of the disclosure.

Referring to FIG. 13, in an embodiment of the disclosure, operations of FIG. 13 may be performed in a server (e.g., the server 108). However, the disclosure is not limited thereto, and at least some of the operations of FIG. 13 may also be performed in the electronic device 401.

In operation 1301, in an embodiment of the disclosure, a server may obtain (e.g., receive) a PWV-and-P_{PWA} pair and a reference blood pressure from each of multiple electronic devices (e.g., multiple electronic devices corresponding to multiple users, respectively) (hereinafter, they are referred to as "multiple electronic devices").

In an embodiment of the disclosure, each of multiple electronic devices (hereinafter, these are referred to as "electronic devices") may obtain a PWV-and-P_{PWA} pair and a reference blood pressure (e.g., a blood pressure measured using a blood pressure monitor (e.g., a blood pressure monitor using a cuff)) (hereinafter, this is referred as a "reference blood pressure") by using a first wearable device (e.g., a smart watch) and a second wearable device (e.g., a smart ring) wirelessly connected to the electronic device. For example, the multiple electronic devices may include a first electronic device and a second electronic device. The first electronic device may perform operation 701 to operation 709 of FIG. 7 described above to obtain a first PWV-and-P_{PWA} pair (e.g., multiple first PWV-and-P_{PWA} pairs) and obtain a first reference blood pressure measured using a blood pressure monitor (e.g., a blood pressure measured using a blood pressure monitor within a designated time before/after a time point at which the first PWV-and-P_{PWA} pair is obtained). The second electronic device may perform operation 701 to operation 709 of FIG. 7 described above to obtain a second PWV-and-P_{PWA} pair (e.g., multiple second PWV-and-P_{PWA} pairs) and obtain a second reference blood pressure measured using a blood pressure monitor (e.g., a blood pressure measured using a blood pressure monitor within a designated time before/after a time point at which the second PWV-and-P_{PWA} pair is obtained).

In an embodiment of the disclosure, the server may receive a PWV-and-P_{PWA} pair (e.g., the first PWV-and-P_{PWA} pair and the second PWV-and-P_{PWA} pair) and a reference blood pressure (e.g., the first reference blood pressure and the second reference blood pressure) from each of the multiple electronic devices through communication circuitry (e.g., communication circuitry of the server).

In operation 1303, in an embodiment of the disclosure, the server may obtain a vascular characteristic value, based on the PWV-and-P_{PWA} pairs and the reference blood pressures obtained from the multiple electronic devices.

In an embodiment of the disclosure, the server may perform, for each of the multiple electronic devices (or the multiple users), a regression analysis using PWV-and-P_{PWA} pairs obtained from the electronic device, to calculate a correlation between PWV² and P_{PWA} (e.g., an equation representing a correlation between PWV² and P_{PWA}). The server may calculate, for each of the multiple electronic devices (or the multiple users), a vascular characteristic vascular (e.g., α and/or γ in [Equation 6] and [Equation 7]) from the calculated correlation between PWV² and P_{PWA}.

For example, the server may perform, for the first electronic device (or a first user of the first electronic device), a regression analysis using multiple PWV-and-PPWA pairs obtained from the first electronic device, to calculate a first correlation between PWV² and P_{PWA} (e.g., an equation representing a correlation between PWV² and P_{PWA}). The server may calculate, for the first electronic device (or the first user), a vascular characteristic vascular (e.g., α and/or γ in [Equation 6] and [Equation 7]), based on the first correlation. The server may perform, for the second electronic device (or a second user of the second electronic device), a regression analysis using multiple PWV-and-PPWA pairs obtained from the second electronic device, to calculate a second correlation between PWV²and P_{PWA} (e.g., an equation representing a correlation between PWV² and P_{PWA}). The server may calculate, for the second electronic device (or the second user), a vascular characteristic vascular (e.g., α and/or γ in [Equation 6] and [Equation 7]), based on the second correlation.

In an embodiment of the disclosure, the server may calculate, for each of the multiple electronic devices (or the multiple users), a blood pressure correction value (e.g., P_{cal} in [Equation 7]), based on P_{PWA} and the reference blood pressure. For example, the server may subtract the first reference blood pressure from P_{PWA} (e.g., P_{PWA} received from the first electronic device) for the first electronic device to calculate a first blood pressure correction value. The server may subtract the second reference blood pressure from P_{PWA} (e.g., P_{PWA} received from the second electronic device) for the second electronic device to calculate a second blood pressure correction value.

In an embodiment of the disclosure, the server may calculate, for each of the multiple electronic devices (or the multiple users), a vascular characteristic value (e.g., β in [Equation 7]), based on a vascular characteristic value (e.g., γ in [Equation 6] and [Equation 7]) and a blood pressure correction value (e.g., P_{cal} in [Equation 7]). For example, the server may input a vascular characteristic value (e.g., γ in [Equation 6] and [Equation 7]) and the first blood pressure correction value (P_{cal}) into [Equation 6] for the first electronic device, to calculate a vascular characteristic value (e.g., β in [Equation 7]). For example, the server may input a vascular characteristic value (e.g., γ in [Equation 6] and [Equation 7]) and the second blood pressure correction value (P_{cal}) into [Equation 6] for the second electronic device, to calculate a vascular characteristic value (e.g., β in [Equation 7]).

In operation 1305, in an embodiment of the disclosure, the server may obtain a correlation between the vascular characteristic value and the blood pressure correction value, based on the vascular characteristic value and the blood pressure correction value.

In an embodiment of the disclosure, the server may perform operation 1303 to obtain multiple vascular characteristic values (e.g., multiple β values) and multiple blood pressure correction values (e.g., P_{cal} values) corresponding to the multiple electronic devices, respectively. For example, the server may perform operation 1303 to obtain the first blood pressure correction value (P_{cal}) and a vascular characteristic value (e.g., β in [Equation 7]) for the first electronic device. The server may perform operation 1303 to obtain the second blood pressure correction value (Pcal) and a vascular characteristic value (e.g., β in [Equation 7]) for the second electronic device.

In an embodiment of the disclosure, the server may perform a regression analysis for multiple vascular characteristic values (e.g., multiple β values) and multiple blood pressure correction values (e.g., P_{cal} values) to calculate a correlation (e.g., an equation or function representing the correlation) (e.g., P_{cal} = f(β)) between a vascular characteristic value (β) and a blood pressure correction value (P_{cal}).

In an embodiment of the disclosure, after the correlation between a vascular characteristic value (β) and a blood pressure correction value (P_{cal}) is calculated, the server may transmit the calculated correlation between a vascular characteristic value (β) and a blood pressure correction value (P_{cal}) to an electronic device (e.g., the electronic device 401). The electronic device (e.g., the electronic device 401) may receive the correlation between a vascular characteristic value (β) and a blood pressure correction value (P_{cal}) through communication circuitry to obtain the correlation between a vascular characteristic value (β) and a blood pressure correction value (P_{cal}). The electronic device may perform operations in FIG. 7 after the correlation between a vascular characteristic value (β) and a blood pressure correction value (P_{cal}) is obtained.

FIG. 13 illustrates an example in which the server performs operation 1301 to operation 1305, but the disclosure is not limited thereto. For example, the electronic device 401 may perform at least some of operation 1301 to operation 1305.

An electronic device 401 according to an embodiment may include communication circuitry 410 and at least one processor 450. The at least one processor 450 may be configured to receive a first PPG signal obtained by a first wearable device 501 from the first wearable device 501 via the communication circuitry 410 and receive a second PPG signal from a second wearable device 601 via the communication circuitry 410. The first wearable device 501 may be worn on a first position of a user and the second wearable device 601 may be worn on a second position of the user. The at least one processor 450 may be configured to obtain a pulse transit time (PTT) during which a pulse wave is transferred from the first position to the second position, based on the first PPG signal and the second PPG signal. The at least one processor 450 may be configured to obtain a distance between the first wearable device 501 and the second wearable device 601. The at least one processor 450 may be configured to obtain a pulse wave velocity (PWV) at which the pulse wave is transferred, based on the distance and the PTT. The at least one processor 450 may be configured to obtain a first blood pressure by using a pulse wave analysis (PWA) method, based on the first PPG signal and/or the second PPG signal. The at least one processor 450 may be configured to obtain a vascular characteristic value related to a characteristic of a blood vessel, based on the PWV and the first blood pressure. The at least one processor 450 may be configured to obtain a blood pressure correction value for correcting a second blood pressure to be obtained, based on the vascular characteristic value.

In an embodiment of the disclosure, the at least one processor 450 may be configured to transmit time information via the communication circuitry 410 so that the first wearable device 501 and the second wearable device 601 are synchronized.

In an embodiment of the disclosure, the at least one processor 450 may be configured to obtain the PTT during which the pulse wave is transferred from the first position to the second position, based on times of mutually corresponding feature points in the first PPG signal and the second PPG signal.

In an embodiment of the disclosure, the electronic device 401 may further include a camera 430. The at least one processor 450 may be configured to transmit, to the first wearable device 501 via the communication circuitry 410, a signal causing the first wearable device 501 to display an object having a designated length. The at least one processor 450 may be configured to, while the first wearable device 501 displays the object, obtain an image for the first wearable device 501 and the second wearable device 601 via the camera 430. The at least one processor 450 may be configured to obtain a first length of a part representing the object in the image, and a first distance between a part representing the first wearable device 501 and a part representing the second wearable device 601 in the image. The at least one processor 450 may be configured to obtain the distance between the first wearable device (501) and the second wearable device (601), based on the designated length of the object, the first length, and the first distance.

In an embodiment of the disclosure, the at least one processor 450 may be configured to obtain the distance between the first wearable device 501 and the second wearable device 601, based on a transmission/reception time of an inaudible sound wave between the first wearable device 501 and the second wearable device 601 and a velocity of the inaudible sound wave.

In an embodiment of the disclosure, the at least one processor 450 may be configured to obtain, by using a PWV blood pressure model and a PWA blood pressure model, a first equation including PWV², the first blood pressure, and a first vascular characteristic value and a second vascular characteristic value representing a correlation between PWV² and the first blood pressure, and obtain a second equation representing a relation between the second vascular characteristic value, a third vascular characteristic value, and the blood pressure correction value.

In an embodiment of the disclosure, the at least one processor 450 may be configured to obtain multiple PWVs and multiple first blood pressures corresponding to the multiple PWVs during a designated period. The at least one processor 450 may be configured to obtain the first vascular characteristic value and the second vascular characteristic value, by using regression analysis, based on the multiple PWVs and the multiple first blood pressures.

In an embodiment of the disclosure, the at least one processor 450 may be configured to obtain the blood pressure correction value by using the second equation and a correlation between the third vascular characteristic value and the blood pressure correction value.

In an embodiment of the disclosure, the at least one processor 450 may be configured to, after obtaining the blood pressure correction value, obtain the second blood pressure by using a PWA method, based on a PPG signal obtained from the first wearable device 501 or the second wearable device 601. The at least one processor 450 may be configured to obtain a third blood pressure by adding the blood pressure correction value to the obtained second blood pressure.

In an embodiment of the disclosure, the electronic device 401 may include a smartphone, the first wearable device 501 may include a smart watch, and the second wearable device 601 may include a smart ring.

A method of measuring a blood pressure by an electronic device 401 according to an embodiment may include receiving a first PPG signal obtained by a first wearable device 501 from the first wearable device 501 via communication circuitry 410 of the electronic device 401, and receiving a second PPG signal from a second wearable device 601 via the communication circuitry 410. The first wearable device 501 may be worn on a first position of a user and the second wearable device 601 may be worn on a second position of the user. The method may include obtaining a pulse transit time (PTT) during which a pulse wave is transferred from the first position to the second position, based on the first PPG signal and the second PPG signal. The method may include obtaining a distance between the first wearable device 501 and the second wearable device 601. The method may include obtaining a pulse wave velocity (PWV) at which the pulse wave is transferred, based on the distance and the PTT. The method may include obtaining a first blood pressure by using a pulse wave analysis (PWA) method, based on the first PPG signal and/or the second PPG signal. The method may include obtaining a vascular characteristic value related to a characteristic of a blood vessel, based on the PWV and the first blood pressure. The method may include obtaining a blood pressure correction value for correcting a second blood pressure to be obtained, based on the vascular characteristic value.

In an embodiment of the disclosure, the method may further include transmitting time information via the communication circuitry 410 so that the first wearable device 501 and the second wearable device 601 are synchronized.

In an embodiment of the disclosure, the obtaining of the PTT may include obtaining the PTT during which the pulse wave is transferred from the first position to the second position, based on times of mutually corresponding feature points in the first PPG signal and the second PPG signal.

In an embodiment of the disclosure, the obtaining of the distance between the first wearable device 501 and the second wearable device 601 may include transmitting, to the first wearable device 501 via the communication circuitry 410, a signal causing the first wearable device 501 to display an object having a designated length. In an embodiment of the disclosure, the obtaining of the distance between the first wearable device 501 and the second wearable device 601 may include, while the first wearable device 501 is displaying the object, obtaining an image for the first wearable device 501 and the second wearable device 601 via a camera 430 of the electronic device 401. The obtaining of the distance between the first wearable device 501 and the second wearable device 601 may include obtaining a first length of a part representing the object in the image, and a first distance between a part representing the first wearable device 501 and a part representing the second wearable device 601 in the image. The obtaining of the distance between the first wearable device 501 and the second wearable device 601 may include obtaining the distance between the first wearable device 501 and the second wearable device 601, based on the designated length of the object, the first length, and the first distance.

In an embodiment of the disclosure, the obtaining of the distance between the first wearable device 501 and the second wearable device 601 may include obtaining the distance between the first wearable device 501 and the second wearable device 601, based on a transmission/reception time of an inaudible sound wave between the first wearable device 501 and the second wearable device 601 and a velocity of the inaudible sound wave.

In an embodiment of the disclosure, the obtaining of the vascular characteristic value related to the characteristic of the blood vessel, based on the PWV and the first blood pressure may include obtaining, by using a PWV blood pressure model and a PWA blood pressure model, a first equation including PWV², the first blood pressure, and a first vascular characteristic value and a second vascular characteristic value representing a correlation between PWV² and the first blood pressure, and obtaining a second equation representing a relation between the second vascular characteristic value, a third vascular characteristic value, and the blood pressure correction value.

In an embodiment of the disclosure, the obtaining of the vascular characteristic value related to the characteristic of the blood vessel, based on the PWV and the first blood pressure may include obtaining multiple PWVs and multiple first blood pressures corresponding to the multiple PWVs during a designated period. The obtaining of the vascular characteristic value related to the characteristic of the blood vessel, based on the PWV and the first blood pressure may include obtaining the first vascular characteristic value and the second vascular characteristic value, by using regression analysis, based on the multiple PWVs and the multiple first blood pressures.

In an embodiment of the disclosure, the obtaining of the blood pressure correction value may include obtaining the blood pressure correction value by using the second equation and a correlation between the third vascular characteristic value and the blood pressure correction value.

In an embodiment of the disclosure, the method may further include, after obtaining the blood pressure correction value, obtaining the second blood pressure by using a PWA method, based on a PPG signal obtained from the first wearable device 501 or the second wearable device 601. The method may further include obtaining a third blood pressure by adding the blood pressure correction value to the second obtained second blood pressure.

In an embodiment of the disclosure, in a non-transitory computer-readable medium for recording computer-executable instructions, the computer-executable instructions may, when executed by at least one processor 450 of an electronic device 401, cause the electronic device 401 to receive a first PPG signal obtained by a first wearable device 501 from the first wearable device 501 via communication circuitry 410 of the electronic device 401 and receive a second PPG signal from a second wearable device 601 via the communication circuitry 410. The first wearable device 501 may be worn on a first position of a user and the second wearable device 601 may be worn on a second position of the user. The computer-executable instructions may, when executed by the at least one processor 450 of the electronic device 401, cause the electronic device 401 to obtain a pulse transit time (PTT) during which a pulse wave is transferred from the first position to the second position, based on the first PPG signal and the second PPG signal. The computer-executable instructions may, when executed by the at least one processor 450 of the electronic device 401, cause the electronic device 401 to obtain a distance between the first wearable device 501 and the second wearable device 601. The computer-executable instructions may, when executed by the at least one processor 450 of the electronic device 401, cause the electronic device 401 to obtain a pulse wave velocity (PWV) at which the pulse wave is transferred, based on the distance and the PTT. The computer-executable instructions may, when executed by the at least one processor 450 of the electronic device 401, cause the electronic device 401 to obtain a first blood pressure by using a pulse wave analysis (PWA) method, based on the first PPG signal and/or the second PPG signal. The computer-executable instructions may, when executed by the at least one processor 450 of the electronic device 401, cause the electronic device 401 to obtain a vascular characteristic value related to a characteristic of a blood vessel, based on the PWV and the first blood pressure. The computer-executable instructions may, when executed by the at least one processor 450 of the electronic device 401, cause the electronic device 401 to obtain a blood pressure correction value for correcting a second blood pressure to be obtained, based on the vascular characteristic value.

In addition, a data structure used in an embodiment disclosed herein may be recorded on a computer-readable recording medium through various means. The computer-readable recording medium may include a storage medium, such as a magnetic storage medium (e.g., ROM, floppy disc, or hard disc) or optical reading medium (e.g., CD-ROM or DVD).

## Claims

1. An electronic device (401) comprising:
communication circuitry (410); and
at least one processor (450),
wherein the at least one processor (450) is configured to:
receive a first photoplethysmography (PPG) signal obtained by a first wearable device (501) from the first wearable device (501) via the communication circuitry (410), and receive a second PPG signal from a second wearable device (601) via the communication circuitry (410), the first wearable device (501) being worn on a first position of a user and the second wearable device (601) being worn on a second position of the user,
based on the first PPG signal and the second PPG signal, obtain a pulse transit time (PTT) during which a pulse wave is transferred from the first position to the second position,
obtain a distance between the first wearable device (501) and the second wearable device (601),
based on the distance and the PTT, obtain a pulse wave velocity (PWV) at which the pulse wave is transferred,
based on the first PPG signal and/or the second PPG signal, obtain a first blood pressure by using a pulse wave analysis (PWA) method,
based on the PWV and the first blood pressure, obtain a vascular characteristic value related to a characteristic of a blood vessel, and
based on the vascular characteristic value, obtain a blood pressure correction value for correcting a second blood pressure to be obtained.

2. The electronic device (401) of claim 1, wherein the at least one processor (450) is configured to transmit time information via the communication circuitry (410) so that the first wearable device (501) and the second wearable device (601) are synchronized.

3. The electronic device (401) of claim 1 or 2, wherein the at least one processor (450) is configured to obtain the PTT during which the pulse wave is transferred from the first position to the second position, based on times of mutually corresponding feature points in the first PPG signal and the second PPG signal.

4. The electronic device (401) of any one of claims 1 to 3, further comprising a camera (430),
wherein the at least one processor (450) is configured to:
transmit, to the first wearable device (501) via the communication circuitry (410), a signal causing the first wearable device (501) to display an object having a designated length,
while the first wearable device (501) displays the object, obtain an image for the first wearable device (501) and the second wearable device (601) via the camera (430),
obtain a first length of a part representing the object in the image, and a first distance between a part representing the first wearable device (501) and a part representing the second wearable device (601) in the image, and
based on the designated length of the object, the first length, and the first distance, obtain the distance between the first wearable device (501) and the second wearable device (601).

5. The electronic device (401) of any one of claims 1 to 4, wherein the at least one processor (450) is configured to obtain the distance between the first wearable device (501) and the second wearable device (601), based on a transmission/reception time of an inaudible sound wave between the first wearable device (501) and the second wearable device (601) and a velocity of the inaudible sound wave.

6. The electronic device (401) of any one of claims 1 to 5, wherein the at least one processor (450) is configured to:
obtain, by using a PWV blood pressure model and a PWA blood pressure model, a first equation including PWV², the first blood pressure, and a first vascular characteristic value and a second vascular characteristic value representing a correlation between PWV² and the first blood pressure, and
obtain a second equation representing a relation between the second vascular characteristic value, a third vascular characteristic value, and the blood pressure correction value.

7. The electronic device (401) of claim 6, wherein the at least one processor (450) is configured to:
obtain, during a designated period, multiple PWVs and multiple first blood pressures corresponding to the multiple PWVs, and
obtain the first vascular characteristic value and the second vascular characteristic value, by using regression analysis, based on the multiple PWVs and the multiple first blood pressures.

8. The electronic device (401) of claim 7, wherein the at least one processor (450) is configured to obtain the blood pressure correction value by using the second equation and a correlation between the third vascular characteristic value and the blood pressure correction value.

9. The electronic device (401) of claim 8, wherein the at least one processor (450) is configured to:
after obtaining the blood pressure correction value, based on a PPG signal obtained from the first wearable device (501) or the second wearable device (601), obtain the second blood pressure by using a PWA method, and
obtain a third blood pressure by adding the blood pressure correction value to the obtained second blood pressure.

10. The electronic device (401) of any one of claims 1 to 9, wherein the electronic device (401) comprises a smartphone,
the first wearable device (501) comprises a smart watch, and
the second wearable device (601) comprises a smart ring.

11. A method of measuring a blood pressure by an electronic device (401), the method comprising:
receiving a first PPG signal obtained by a first wearable device (501) from the first wearable device (501) via communication circuitry (410) of the electronic device (401), and receiving a second PPG signal from a second wearable device (601) via the communication circuitry (410), the first wearable device (501) being worn on a first position of a user and the second wearable device (601) being worn on a second position of the user;
based on the first PPG signal and the second PPG signal obtaining a pulse transit time (PTT) during which a pulse wave is transferred from the first position to the second position;
obtaining a distance between the first wearable device (501) and the second wearable device (601);
based on the distance and the PTT, obtaining a pulse wave velocity (PWV) at which the pulse wave is transferred;
based on the first PPG signal and/or the second PPG signal, obtaining a first blood pressure by using a pulse wave analysis (PWA) method;
based on the PWV and the first blood pressure, obtaining a vascular characteristic value related to a characteristic of a blood vessel; and
based on the vascular characteristic value, obtaining a blood pressure correction value for correcting a second blood pressure to be obtained.

12. The method of claim 11, further comprising transmitting time information via the communication circuitry (410) so that the first wearable device (501) and the second wearable device (601) are synchronized.

13. The method of claim 11 or 12, wherein the obtaining of the PTT comprises obtaining the PTT during which the pulse wave is transferred from the first position to the second position, based on times of mutually corresponding feature points in the first PPG signal and the second PPG signal.

14. The method of any one of claims 11 to 13, wherein the obtaining of the distance between the first wearable device (501) and the second wearable device (601) comprises:
transmitting, to the first wearable device (501) via the communication circuitry (410), a signal causing the first wearable device (501) to display an object having a designated length;
while the first wearable device (501) displays the object, obtaining an image for the first wearable device (501) and the second wearable device (601) via a camera (430) of the electronic device (401);
obtaining a first length of a part representing the object in the image, and a first distance between a part representing the first wearable device (501) and a part representing the second wearable device (601) in the image; and
based on the designated length of the object, the first length, and the first distance, obtaining the distance between the first wearable device (501) and the second wearable device (601).

15. The method of any one of claims 11 to 14, wherein the obtaining of the distance between the first wearable device (501) and the second wearable device (601) comprises obtaining the distance between the first wearable device (501) and the second wearable device (601), based on a transmission/reception time of an inaudible sound wave between the first wearable device (501) and the second wearable device (601) and a velocity of the inaudible sound wave.
